# EUROPEAN PATENT APPLICATION

(11) **EP 4 660 326 A2**
(43) Date of publication of application: **10.12.2025**
(21) Application number: 25204930.9
(22) Date of filing: 13.11.2020
(51) Int. Cl.: C12Q 1/70

(54) **IDENTIFICATION OF HOST RNA BIOMARKERS OF INFECTION**

(30) Priority: 13.11.2019 US 201962934873 P; 07.04.2020 US 202063006561 P
(62) Divisional of application: 24194139.2
(71) Applicant: The Regents of the University of Colorado, a body corporate, Denver, CO 80203 (US)
(72) Inventor: SAWYER, Sara L., Boulder, CO 90302 (US); DOWELL, Robin, Broomfield, CO 80020 (US); YANG, Qing, Longmont, CO 80501 (US); MEYERSON, Nicholas R., Broomfield, CO 80020 (US)
(74) Representative: Berggren Oy

(57) **Abstract**

The inventive technology includes novel systems, method and compositions for the identification and classification of host-derived RNA biomarkers produced in response to an infection.

## Description

### CROSS-REFERENCE TO RELATED APPLICATIONS

This International PCT Application claims the benefit of and priority to U.S. Provisional Application No. 62/934,873, filed November 13, 2019, and U.S. Provisional Application No. 63/006,561, filed April 7, 2020, both of which are incorporated herein by reference in their entirety.

### STATEMENT OF FEDERALLY SPONSORED RESEARCH

This invention was made with government support under grant number HDTRA1-18-1-0032 awarded by DOD/DTRA. The government has certain rights in the invention.

### SEQUENCE LISTING

The instant application contains a Sequence Listing which has been submitted electronically in ASCII format and is hereby incorporated by reference in its entirety. Said ASCII copy, created on November 13, 2020, is named "90245-00442-Sequence-Listing-AF.txt" and is 116 Kbytes in size.

### TECHNICAL FIELD

The inventive technology includes novel systems, method and compositions for the identification and correlation of host-derived RNA biomarkers produced in response to an infection.

### BACKGROUND

Early detection of infection by pathogenic microorganisms is vital for proper treatment and positive clinical outcomes. However, infected individuals may remain asymptomatic for several days post-infection while actively transmitting the pathogen to others. As opposed to the specialized, and later developing adaptive immune response, a host's first line of defense against pathogenic microorganisms is the "innate immune" response (including but not exclusive to the interferon response). The body's innate immunity is a self-amplifying and non-specific physiological response that occurs within hours of infection while the host may be asymptomatic. For example, as part of a host's innate immune response, the human body turns on the expression of specific genes and noncoding RNAs that help in immune defense in response to a bacterial or viral infection.

The expression of these early innate immunity response genes and noncoding RNAs can also serve as a valuable early diagnostics signature that would allow one to: (1) detect that a human has contracted a viral or bacterial infection, and 2) infer some information about the nature of the infection. The ability to detect the presence of molecules produced by a host's innate immune response, and compare those to known host-derived biomarkers that may further be specific for a specific type of infection, while a patient is still asymptomatic may allow effective quarantine protocols, as well as improved treatment and clinical outcomes.

As such, there exists a long-felt need for an effective system to identify and classify host infection biomarkers, and preferably early pre-clinical host RNA biomarkers produced by the body's innate immune system such that early diagnosis and treatment protocols may be more effectively implemented.

### SUMMARY OF THE INVENTION

In one aspect, the invention includes systems and methods to identify host-derived biomarkers, and preferably RNA biomarkers of infection. In one preferred aspect, the invention's system combines multiple statistical models to combine the differential expression analysis results from individual studies to identify and classify biomarkers, and preferably RNA biomarkers of infection. Additional aspects include systems and methods for *in silico* validation and filtering of biomarkers, and preferably RNA biomarkers of infection, that involves using identified biomarkers as classification criteria to determine if a given sample is infected.

In one aspect, the invention includes a bioinformatics-based pipeline configured to identify RNA biomarkers that are indicative of host response to specific infection type. In one preferred aspect, the invention includes a bioinformatics-based pipeline configured to classify RNA biomarkers that are indicative of a host response to a specific type of infection. In this preferred aspect, the invention's novel bioinformatics-based pipeline may be specifically configured to identify host RNA biomarkers may be further classified to differentiate a host response that is specific to viral, or bacterial, infection.

In another aspect, the invention may include a bioinformatics-based pipeline configured to identify host RNA biomarkers that are infection-specific. For example, in this aspect, the infection-specific biomarkers may be identified and classified to differentiate host response that is specific to one or more pathogen classes, such as retrovirus or herpesvirus pathogens.

In another aspect, the invention may include a bioinformatics-based pipeline configured to identify host RNA biomarkers that are infection site, or tissue specific. For example, in this aspect, the infection-specific biomarkers may be identified and classified to differentiate host response that is specific to one or more infection locations, such as a respiratory infection in the host's lungs and/or airway, or in the host's blood.

In another aspect, the invention may include one or more of the host-biomarkers comprising nucleotide sequences identified in: SEQ ID NOs. 1-30. In another aspect, the invention may include one or more virus-specific host RNA biomarkers comprising nucleotide sequences identified in: SEQ ID NOs. 1-5. In another aspect, the invention may include one or more retrovirus-specific host RNA biomarkers comprising nucleotide sequences identified in SEQ ID NOs. 6-10. In another aspect, the invention may include one or more herpesvirus host RNA biomarkers comprising nucleotide sequences identified in: SEQ ID NOs. 11-15. In another aspect, the invention may include one or more respiratory virus-specific host RNA biomarkers comprising nucleotide sequences identified in: SEQ ID NOs. 16-20. In another aspect, the invention may include one or more bacteria-specific host RNA biomarkers comprising nucleotide sequences identified in: SEQ ID NOs. 21-25. In another aspect, the invention may include one or more eukaryotic pathogen-specific host RNA biomarkers comprising nucleotide sequences identified in: SEQ ID NOs. 26-30.

In another aspect, the invention may include the diagnostic use of one or more of the host-biomarkers comprising nucleotide sequences identified in: SEQ ID NOs. 1-30. In another aspect, one or more of the nucleotide sequences identified in SEQ ID NOs. 1-30, and their corresponding encoded mRNA transcript and or translated polypeptide may be used as biomarkers for early-infection in a subject. In another aspect, one or more of the nucleotide sequences identified in SEQ ID NOs. 1-30, and their corresponding encoded mRNA transcript and or translated polypeptide may be used as biomarkers for identification of the site of replication, or infection in a subject. In another aspect, one or more of the nucleotide sequences identified in SEQ ID NOs. 1-30, and their corresponding encoded mRNA transcript and or translated polypeptide may be used as biomarkers for identification of pathogen class-specific infection in a subject.

Additional aspects of the invention may be evidenced from the specification, claims and figures provided below.

### BRIEF DESCRIPTION OF DRAWINGS

The novel aspects, features, and advantages of the present disclosure will be better understood from the following detailed descriptions taken in conjunction with the accompanying figures, all of which are given by way of illustration only, and are not limiting the presently disclosed embodiments, in which:

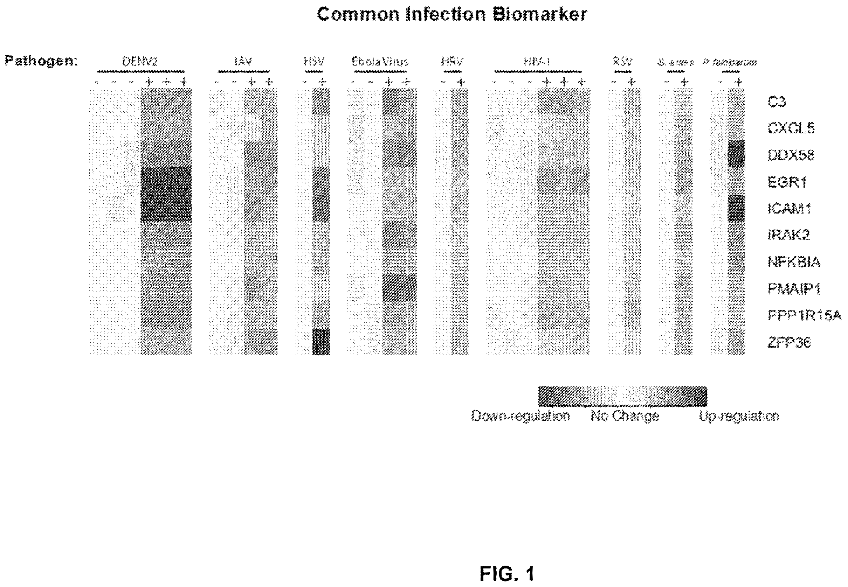

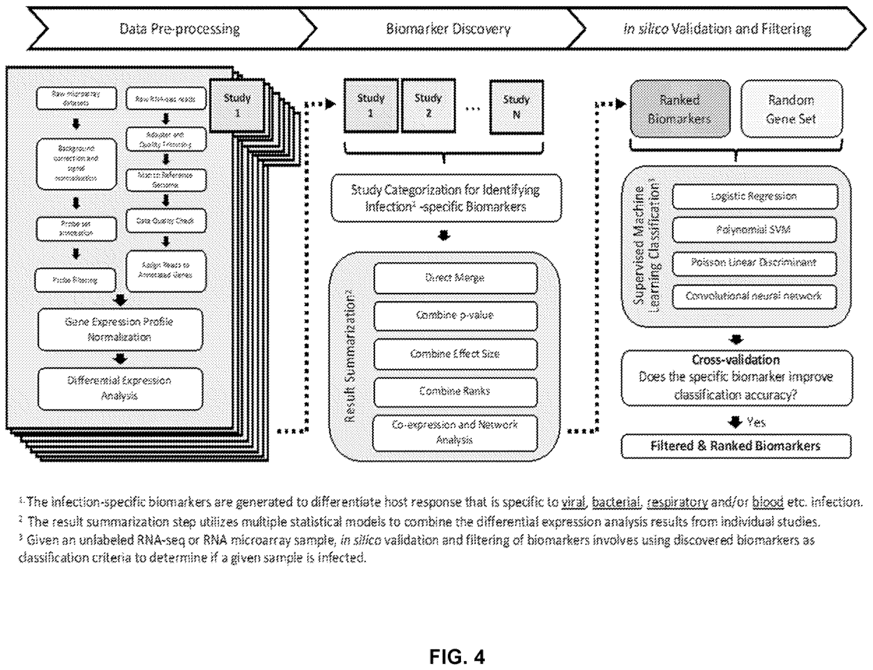

**FIG. 1****:** shows 15 host-derived RNA biomarkers that are consistently upregulated during infection by various pathogens. In one embodiment, such host-derived RNA biomarkers may be "general" biomarkers of infection. Previously published RNA sequencing and microarray data curated from public-domain databases and was analyzed using the bioinformatic pipeline illustrated in FIG. 4 below. Vertically, the top 10 host biomarkers are shown and, horizontally, 8 of the studies that carried out infection using 9 different pathogens were chosen for demonstration. In each study, (-) columns indicate mock-infected cells, while (+) indicate infected cells. All expression level of the biomarkers are relative to the mock infection control, red indicates upregulation of that specific biomarker after infection, blue indicates downregulation, see scale at bottom. Biomarkers were identified and ranked based on how consistently they were upregulated during infection by various pathogens (discussed below and FIG. 4). DENV2 = dengue virus type 2; IAV = influenza A virus; HSV = herpes simplex virus; HRV = human rhinovirus; RSV = respiratory syncytial virus. All are viral pathogens except for *S. aureus* which is a bacterial pathogen, and , and *Plasmodium falciparum*, which is an exemplary eukaryote pathogen.

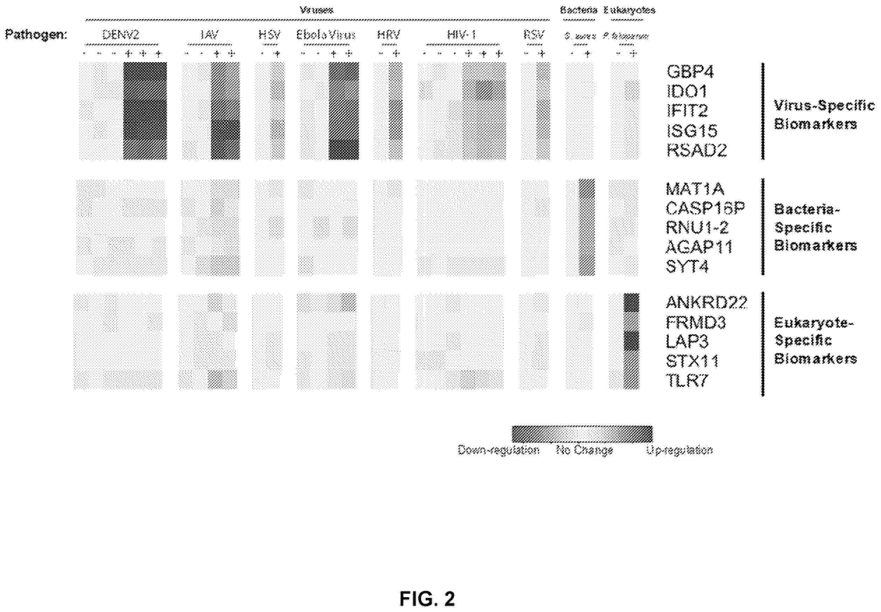

**FIG. 2****:** Certain RNA biomarkers may differentiate between different types of pathogen infection, for example eukaryotic or bacterial versus viral infection. RNA sequencing and microarray datasets (described in the legend to FIG. 1) were further divided into viral versus bacterial and eukaryotic infections. Each subset of data was then analyzed using the biomarker identification pipeline discussed below (and FIG. 4). Biomarkers that are distinctive among viral/bacterial/eukaryotic infection were selected. This embodiment allows the present inventors to distinguish infection origin using host biomarkers. All biomarker expression levels are relative to the mock infection control, red indicates upregulation of that specific biomarker after infection, blue indicates downregulation.

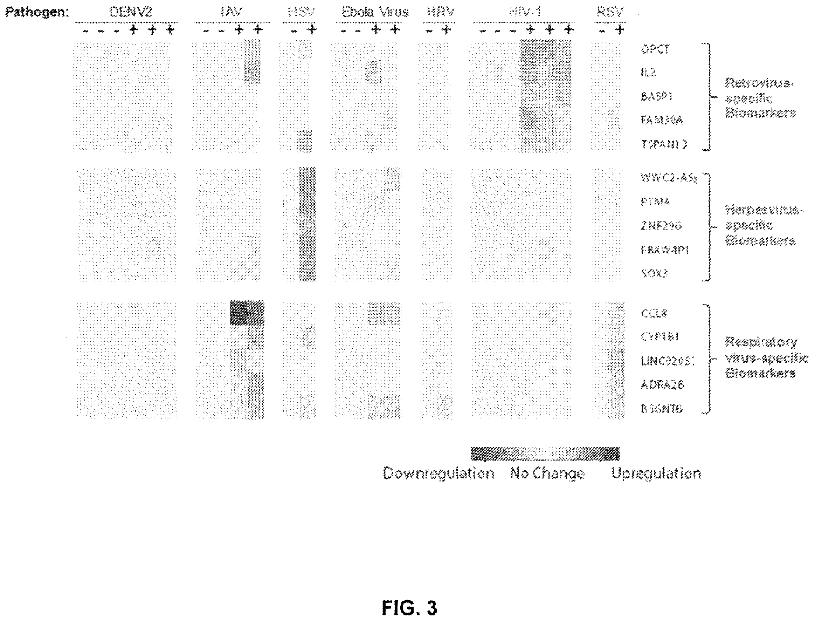

**FIG. 3****:** Biomarkers that identify infection by different categories of viruses or sites of replication in the human body. RNA sequencing and microarray datasets (described above in FIG. 1 legend) were further divided into different virus categories (here, HIV-1 retrovirus or HSV herpesvirus) or sites of pathogen replication in the human body (here, respiratory viruses). This allows us to further define the nature of the infection using specific host-derived biomarkers of infection. All expression level of the biomarkers is relative to the mock infection control, red indicates upregulation of that specific biomarker after infection, blue indicates downregulation.
**FIG. 4****:** Generalized schematic of bioinformatics pipeline used to identify RNA biomarkers that are indicative of host response to specific infection. High-throughput RNA sequencing (RNA-seq) data or RNA microarray data of host response to infection is may be generated, for example by performing qRT-PCR or microarray assays on one or more biological samples that may contain one or more host derived biomarkers, or alternatively curated from publicly accessible databases (NCBI SRA, NCBI GEO). Each RNA-seq or microarray dataset may be generated by different studies. The collection includes multiple cell types and human samples that are infected by different pathogens, including RNA and DNA viruses, and various bacteria species. Additional *in vitro* and *in vivo* infection studies may also be carried out to validate and/or generate more reference datasets. In one embodiment, infection-specific biomarkers are generated to differentiate host response that is specific to viral, bacterial, respiratory and/or blood etc. infection. The result summarization step utilizes multiple statistical models to combine the differential expression analysis results from individual studies. Given an unlabeled RNA-seq sample, *in silico* validation and filtering of biomarkers involves using discovered biomarkers as classification criteria to determine if a given sample is infected.

### DETAILED DESCRIPTION OF INVENTION

In one embodiment, the invention includes systems, methods and compositions for the identification and classification of host biomarkers produced in response to an infection. In one preferred embodiment, the invention includes systems, methods and compositions for the identification and classification of early RNA biomarkers produced by the cell or subjects innate immune response in response to an infection. Notably, such specific target RNA transcripts or biomarkers produced by a patient's innate immune response may be indicative of early infection. As a result, in one embodiment of the inventive technology may include systems, methods and compositions for the detection of these target RNA transcripts which may act as biomarkers for early-infection in a subject.

In one preferred embodiment of the invention, to identify host-derived RNA biomarkers of infection, cells in culture or in a subject, such as a human subject, may be infected with various pathogens and then the RNA of the cell or tissues, and preferably mammalian tissues, and more preferably human tissue is collected and sequenced and compared to a (-) infection control. When different conditions and pathogens are compared to each other, general host RNA biomarkers can be initially derived as shown specifically in FIG. 1, red boxes indicates that a host gene is upregulated in response to the infection challenge. In a preferred embodiment of the inventive technology, the present inventor may specifically identify universally upregulated genes like EGR1, that are turned on in all or most infections tested. Such general host RNA biomarkers may be diagnostically indicative of a variety of different type and sites of infection in a subject and may further be used to generate an initial non-specific diagnosis of an early infection in a subject.

In another preferred embodiment of the invention, the RNA biomarkers produced by the host in response to an infection challenge may be compared between different classes of pathogens. In this manner, specific biomarkers, and preferably host-derived RNA biomarkers, can be identified and classified to indicate different types of infection. For instance, in one embodiment shown in FIG. 2, the present inventors identified biomarkers that differentiate bacterial versus viral infection. In another example shown in FIG. 3, the present inventive technology can be used to identify host-derived biomarkers, and preferably host-derived RNA biomarkers, that are specific to different classes of pathogens (*e.g*. retroviruses, or herpesviruses), or different sites of pathogen replication in the body (*e.g*. respiratory, or gastrointestinal viruses). As outlined in FIG. 4, through *in silico* validation, the present inventors can employ computer-assisted processes to confirm that each of these sets of biomarkers reliably detect and differentiate viral versus bacterial infection; retrovirus versus other infection and the like.

Alternately, in another embodiment, the target biomarkers can be empirically tested in human or other *in vivo* trials. For example, one embodiment of the invention includes the validation of target RNA biomarkers of infection using quantitative reverse transcription polymerase chain reaction (RT-PCR) protocols. As biomarkers identified using the methods outlined above may be further confirmed in tissue culture infection experiments. Quantitative RT-PCR (qRT-PCR) of RNA allows specific quantification of the upregulation of candidate biomarkers as a 'fold change' in infected cells compared to uninfected cells. Such information helps when evaluating detection sensitivity with respect to a given biomarker. While only twenty-five exemplary biomarker candidates are being identified herein, such list should not be construed as limiting on the number of biomarkers that may identified with the current invention.

As further highlighted in FIG. 4, high-throughput RNA sequencing (RNA-seq) data as well as quantitative RNA microarray data of the host response to infection may curated from publicly accessible databases (e.g., NCBI SRA, NCBI GEO) or created in house using *in vitro or in vivo* infection challenge experiments, or both to generate biomarker datasets for analysis and identification. Each RNA-seq or RNA microarray dataset may preferably be derived from human cells or tissues that have been infected with one or more pathogen, and then the human RNA response is probed and quantified. A mock (- infection) control or healthy tissue samples may be used in order to subtract out the RNA biomarkers that were already being produced in the cells before they were infected. Notably, as highlighted above, that while it might seem counter-intuitive to combine datasets from different labs, this can also be of benefit. When RNA-seq and RNA microarray datasets are generated by different groups, in different human cell lines or tissues, using different pathogens, and under different conditions, then any host-derived RNA biomarkers of infection upregulated in all of these datasets (*see e.g.*, FIG. 1) has a high probability of being a robust general biomarker.

In one embodiment the invention may include systems, methods and compositions for the identification and use of one or more host-derived RNA biomarkers of infection. In one preferred embodiment, a first tissue culture experiment can be established and tested to identify target RNA transcripts that may be upregulated during an experimental infection, and that may also be secreted from target cells. RNAs that are upregulated may be used as candidate biomarkers and engineered for compatibility with biomarker detection systems, such as the lateral flow device, as well as qRT-PCR methods and systems generally described by the present inventors in US PCT Application No. PCT/US2020/049290, the specification, figures and sequence identification being incorporated herein by reference. In parallel, RNAs from healthy and infected human saliva may be characterized in a clinical trial (right) in order to identify RNA biomarkers of infection in humans. Those biomarkers, if not already identified in the tissue culture experiments, may be engineered for compatibility with the lateral flow system as generally describe above.

In another embodiment, the invention may include one or more of the host-biomarkers comprising nucleotide sequences identified in: SEQ ID NOs. 1-30. In another embodiment, the invention may include one or more virus-specific host RNA biomarkers comprising nucleotide sequences identified in: SEQ ID NOs. 1-5. In another embodiment, the invention may include one or more retrovirus-specific host RNA biomarkers comprising nucleotide sequences identified in SEQ ID NOs. 6-10. In another embodiment, the invention may include one or more herpesvirus host RNA biomarkers comprising nucleotide sequences identified in: SEQ ID NOs. 11-15. In another embodiment, the invention may include one or more respiratory virus-specific host RNA biomarkers comprising nucleotide sequences identified in: SEQ ID NOs. 16-20. In another embodiment, the invention may include one or more eukaryotic pathogen-specific host RNA biomarkers comprising nucleotide sequences identified in: SEQ ID NOs. 16-20.

In another embodiment, the invention may include one or more bacteria-specific host RNA biomarkers comprising nucleotide sequences identified in: SEQ ID NOs. 1-30. In another embodiment, the invention may include the diagnostic use of one or more of the host-biomarkers comprising nucleotide sequences identified in: SEQ ID NOs. 1-30. In one another embodiment, a of one or more of the nucleotide sequences identified in SEQ ID NOs. 1-30, and their corresponding encoded mRNA transcript and or translated polypeptide may be used as biomarkers for early-infection in a subject. In one another embodiment, a of one or more of the nucleotide sequences identified in SEQ ID NOs. 1-30, and their corresponding encoded mRNA transcript and or translated polypeptide may be used as biomarkers for identification of the site of replication, or infection in a subject. In one another embodiment, a of one or more of the nucleotide sequences identified in SEQ ID NOs. 1-30, and their corresponding encoded mRNA transcript and or translated polypeptide may be used as biomarkers for identification of pathogen class-specific infection in a subject.

In another embodiment, identification of one or more RNA biomarkers of infection may help inform treatment of a subject. For example, identification of viral or bacterial-specific host RNA biomarkers may guide a medical practitioner to administer an anti-viral or an antibiotic. It may also, in the case of a viral infection such as SARS-CoV-2, guide a medical practitioner to recommend the subject be quarantined. For example, identification of viral RNA biomarkers associated with a respiratory infection may guide a medical practitioner to administer treatments appropriate for a viral respiratory infection.

The terminology used herein is for describing embodiments and is not intended to be limiting. As used herein, the singular forms "a," "and" and "the" include plural referents, unless the content and context clearly dictate otherwise. Thus, for example, a reference to "a biomarker" may include a combination of two or more such biomarkers. Unless defined otherwise, all scientific and technical terms are to be understood as having the same meaning as commonly used in the art to which they pertain. As used herein, "about" or "approximately" means within 10% of a stated concentration range or within 10% of a stated time frame.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

Nucleic acids and/or other moieties of the invention may be isolated. As used herein, "isolated" means separate from at least some of the components with which it is usually associated whether it is derived from a naturally occurring source or made synthetically, in whole or in part. Nucleic acids and/or other moieties of the invention may be purified. As used herein, purified means separate from the majority of other compounds or entities. A compound or moiety may be partially purified or substantially purified. Purity may be denoted by weight measure and may be determined using a variety of analytical techniques such as but not limited to mass spectrometry, HPLC, etc.

As used herein, a biological marker ("biomarker" or "marker") is a characteristic that is objectively measured and evaluated as an indicator of normal biologic processes, pathogenic processes, or pharmacological responses to therapeutic interventions, consistent with NIH Biomarker Definitions Working Group (1998). Markers can also include patterns or ensembles of characteristics indicative of particular biological processes. The biomarker measurement can increase or decrease to indicate a particular biological event or process. In addition, if the biomarker measurement typically changes in the absence of a particular biological process, a constant measurement can indicate occurrence of that process. In a preferred embodiment an RNA biomarker of infection, includes one or more RNA transcripts that may be indicative of infection or other normal or abnormal physiological process. It should be noted that where RNA biomarker of infection is referenced, it includes the sequence of the RNA transcript, whether of the DNA or mRNA sequence, as well as all alternatively spliced RNA transcripts or RNA biomarkers of infection that have undergone an alternative splicing event, as well as related polynucleotides.

The term "alternative splicing event", as used herein, designates any sequence variation existing between two polynucleotide arising from the same gene or the same pre-mRNA by alternative splicing. This term also refers to polynucleotides, including splicing isoforms or fragments thereof, comprising said sequence variation. Preferably, said sequence variation is characterized by an insertion or deletion of at least one exon or part of an exon. The term "alternative splicing events" encompasses the original alternative splicing events, the skipping of exon (Dietz et al. , Science 259, 680 (1993); Liu et al., Nature Genet. 16, 328-329 (1997); Nystrom-Lahti et al. Genes Chromosomes Cancer 26: 372-375 (1999)), differential splicing due to the cellular environmental conditions (e.g. cell type or physical stimulus) or to a mutation leading to abnormalities of splicing (Siffert et al., Nature Genetics 18: 45-48 (1998)).

The term "related polynucleotides", as used herein, refers to polynucleotides having identical sequences except for one or a small number of regions that either have a different sequence, or are deleted or added from one polynucleotide compared to the other. Typical related polynucleotides are splicing isoforms of a same gene, or a gene harboring a genomic deletion or addition compared to another allele of the same gene. Such related polynucleotides may be either full-length polynucleotides such as genomic DNA, mRNAs, full-length cDNAs, or fragments thereof.

As referred to herein, the terms "nucleic acid", "nucleic acid molecules" "oligonucleotide", "polynucleotide", and "nucleotides" may interchangeably be used. The terms are directed to polymers of deoxyribonucleotides (DNA), ribonucleotides (RNA), and modified forms thereof in the form of a separate fragment or as a component of a larger construct, linear or branched, single stranded, double stranded, triple stranded, or hybrids thereof. The term also encompasses RNA/DNA hybrids. The polynucleotides may include sense and antisense oligonucleotide or polynucleotide sequences of DNA or RNA. The DNA molecules may be, for example, but not limited to: complementary DNA (cDNA), genomic DNA, synthesized DNA, recombinant DNA, or a hybrid thereof. The RNA molecules may be, for example, but not limited to: ssRNA or dsRNA and the like. The terms further include oligonucleotides composed of naturally occurring bases, sugars, and covalent internucleoside linkages, as well as oligonucleotides having non-naturally occurring portions, which function similarly to respective naturally occurring portions. The terms "nucleic acid segment" and "nucleotide sequence segment," or more generally "segment," will be understood by those in the art as a functional term that includes both genomic sequences, ribosomal RNA sequences, transfer RNA sequences, messenger RNA sequences, operon sequences, and smaller engineered nucleotide sequences that are encoded or may be adapted to encode, peptides, polypeptides, or proteins. Further, it should be noted that when any sequence is referenced herein, for example a DNA sequence, the corresponding RNA and amino acid sequence is also specifically encompassed in such a disclosure.

As referred to herein, the term "database" is directed to an organized collection of biological sequence information and/or quantitative measurement of gene expression that may be stored in a digital form. They specifically include open source, as well as non-open source databases. In some embodiments, the database may include any sequence information. In some embodiments, the database may include the genome sequence of a subject or a microorganism. In some embodiments, the database may include expressed sequence information, such as, for example, an EST (expressed sequence tag) or cDNA (complementary DNA) databases. In some embodiments, the database may include non-coding sequences (that is, untranslated sequences), such as, for example, the collection of RNA families (Rfam) which contains information about non-coding RNA genes, structured cis-regulatory elements and self-splicing RNAs. In some embodiments, the databases may include quantitative measurement of expressed gene abundance, such as, for example, the collection of RNA, DNA or cDNA microarray readout. In some embodiments, the databases may include a collection of cDNA sequences captured from biological samples undergoing specific treatment conditions. Such collection of cDNA sequences can be analyzed to determine the relative abundance of gene expressed in the given biological samples, such as, for example, the collection of RNA sequencing data. In exemplary embodiments, the databases may be selected from redundant or non-redundant NCBI SRA database (which is NIH short read sequencing archive database containing publicly available RNA-seq datasets), NCBI GEO database (which is NIH gene expression omnibus database containing publicly available microarray database), NCBI BioProject database (NIH database containing metadata of experimental setup, protocol, patient information etc. relevant to datasets available on NCBI SRA and GEO databases), GenBank databases (which are the NIH genetic sequence database, an annotated collection of all publicly available DNA and RNA sequences). In exemplary embodiments, the databases may be selected from NCBI Short Read Archive databases. Exemplary databases may be selected from, but not limited to: GenBank CDS (Coding sequences database), PDB (protein database), SwissProt database, PIR (Protein Information Resource) database, PRF (protein sequence) database, EMBL Nucleotide Sequence database, NCBI BioProject database, NCBI SRA (Short Read Archive) database, NCBI GEO (Gene Expression Omnibus) database, Broad Institute GTEx (Genotype-Tissue Expression) database, EMBL Expression Atlas, and the like, or any combination thereof.

As used herein, the term "detection" refers to the qualitative determination of the presence or absence of a microorganism in a sample. The term "detection" also includes the "identification" of a microorganism, i.e., determining the genus, species, or strain of a microorganism according to recognized taxonomy in the art and as described in the present specification. The term "detection" further includes the quantitation of a microorganism in a sample, e.g., the copy number of the microorganism in a microliter (or a milliliter or a liter) or a microgram (or a milligram or a gram or a kilogram) of a sample. The term "detection" also includes the identification of an infection in a subject or sample.

As used herein the term "pathogen" refers to an organism, including a microorganism, which causes disease in another organism (e.g., animals and plants) by directly infecting the other organism, or by producing agents that causes disease in another organism (e.g., bacteria that produce pathogenic toxins and the like). As used herein, pathogens include, but are not limited to bacteria, protozoa, fungi, nematodes, viroids and viruses, or any combination thereof, wherein each pathogen is capable, either by itself or in concert with another pathogen, of eliciting disease in vertebrates including but not limited to mammals, and including but not limited to humans. The term also specifically includes eukaryotic or protist pathogens, such as the Plasmodium sp. that are the causative agent of Malaria. As used herein, the term "pathogen" also encompasses microorganisms which may not ordinarily be pathogenic in a non-immunocompromised host.

As used herein, the step of introducing a pathogen to a subject may include both the intentional introduction of a pathogen, such as through a clinical trial, or through the natural and unintended introduction of a pathogen that may have been introduced to a subject, for example, through an horizontal or vertical pathogen exposure, as well as direct and indirect pathogen transmission, for example including, but not limited to environmental exposure to a pathogen, zoonotic exposure to a pathogen, vector-borne exposure to a pathogen. nosocomial exposure to a pathogen.

The term "infection" or "infect" as used herein is directed to the presence of a microorganism within a subject body and/or a subject cell. For example, a virus may be infecting a subject cell. A parasite (such as, for example, a nematode) may be infecting a subject cell/body. In some embodiments, the microorganism may comprise a virus, a bacteria, a fungi, a parasite, or combinations thereof. According to some embodiments the microorganism is a virus, such as, for example, dsDNA viruses (such as, for example, Adenoviruses, Herpesviruses, Poxviruses), ssDNA viruses (such as, for example, Parvoviruses), dsRNA viruses (such as, for example, Reoviruses), (+) ssRNA viruses (+) sense RNA (such as, for example, Picornaviruses, Togaviruses), (-) ssRNA viruses (-) sense RNA (such as, for example, Orthomyxoviruses, Rhabdoviruses), ssRNA-RT viruses (+) sense RNA with DNA intermediate in life-cycle (such as, for example, Retroviruses), dsDNA-RT viruses (such as, for example, Hepadnaviruses). In some embodiments, the microorganism is a bacteria, such as, for example, a gram negative bacteria, a gram positive bacteria, and the like. In some embodiments, the microorganism is a fungi, such as yeast, mold, and the like. In some embodiments, the microorganism is a parasite, such as, for example, protozoa and helminths or the like. In some embodiments, the infection by the microorganism may inflict a disease and/or a clinically detectable symptom to the subject. In some embodiments, infection by the microorganism may not cause a clinically detectable symptom. In some embodiments, the microorganism is a symbiotic microorganism. In additional embodiments, the microorganism may comprise archaea, protists; microscopic plants (green algae), plankton, and the planarian. In some embodiments, the microorganism is unicellular (single-celled). In some embodiments, the microorganism is multicellular.

As used herein, the term "asymptomatic" refers to an individual who does not exhibit physical symptoms characteristic of being infected with a given pathogen, or a given combination of pathogens.

The target biomarkers of this invention may be used for diagnostic and prognostic purposes, as well as for therapeutic, drug screening and patient stratification purposes (e.g., to group patients into a number of "subsets" for evaluation), as well as other purposes described herein.

Some embodiments of the invention comprise detecting in a sample from a patient, a level of a biomarker, wherein the presence or expression levels of the biomarker are indicative of infection or possible infection by one or more pathogens. As used herein, the term "biological sample" or "sample" includes a sample from any bodily fluid or tissue. Biological samples or samples appropriate for use according to the methods provided herein include, without limitation, blood, serum, urine, saliva, tissues, cells, and organs, or portions thereof. A "subject" is any organism of interest, generally a mammalian subject, and preferably a human subject.

As noted above, in one embodiment qRT-PCR may be utilized to identify one or more host-derived biomarkers of infection. In certain embodiment, intercalator dyes may be used to measure the accumulation of both specific and nonspecific PCR products when utilizing RT-PCR products. For example, intercalator dyes such as SYBR green and TaqMan may be used to detect and identify host-derived biomarkers of infection in a qRT-PCR assay.

Any isothermal amplification protocol can be used according to the methods provided herein. Exemplary types of isothermal amplification include, without limitation, nucleic acid sequence-based amplification (NASBA), loop-mediated isothermal amplification (LAMP), strand displacement amplification (SDA), helicase-dependent amplification (HDA), nicking enzyme amplification reaction (NEAR), signal mediated amplification of RNA technology (SMART), rolling circle amplification (RCA), isothermal multiple displacement amplification (EVIDA), single primer isothermal amplification (SPIA), recombinase polymerase amplification (RPA), and polymerase spiral reaction (PSR, available at nature.com/articles/srepl2723 on the World Wide Web). In some cases, a forward primer is used to introduce a T7 promoter site into the resulting DNA template to enable transcription of amplified RNA products via T7 RNA polymerase. In other cases, a reverse primer is used to add a trigger sequence of a toehold sequence domain.

As used herein, the term "amplified" refers to polynucleotides that are copies of a particular polynucleotide, produced in an amplification reaction. An amplified product, according to the invention, may be DNA or RNA, and it may be double-stranded or single-stranded. An amplified product is also referred to herein as an "amplicon". As used herein, the term "amplicon" refers to an amplification product from a nucleic acid amplification reaction. The term generally refers to an anticipated, specific amplification product of known size, generated using a given set of amplification primers.

Naturally as can be appreciated, all of the steps as herein described may be accomplished in some embodiments through any appropriate machine and/or device resulting in the transformation of, for example data, data processing, data transformation, external devices, operations, and the like. It should also be noted that in some embodiments, software and/or software solution may be utilized to carry out the objectives of the invention and may be defined as software stored on a magnetic or optical disk or other appropriate physical computer readable media including wireless devices and/or smart phones. In alternative embodiments the software and/or data structures can be associated in combination with a computer or processor that operates on the data structure or utilizes the software. Further embodiments may include transmitting and/or loading and/or updating of the software on a computer perhaps remotely over the internet or through any other appropriate transmission machine or device, or even the executing of the software on a computer resulting in the data and/or other physical transformations as herein described.

Certain embodiments of the inventive technology may utilize a machine and/or device which may include a general purpose computer, a computer that can perform an algorithm, computer readable medium, software, computer readable medium continuing specific programming, a computer network, a server and receiver network, transmission elements, wireless devices and/or smart phones, internet transmission and receiving element; cloud-based storage and transmission systems, software updateable elements; computer routines and/or subroutines, computer readable memory, data storage elements, random access memory elements, and/or computer interface displays that may represent the data in a physically perceivable transformation such as visually displaying said processed data. In addition, as can be naturally appreciated, any of the steps as herein described may be accomplished in some embodiments through a variety of hardware applications including a keyboard, mouse, computer graphical interface, voice activation or input, server, receiver and any other appropriate hardware device known by those of ordinary skill in the art.

As used herein, a machine learning system or model is a trained computational model that takes a feature of interest, such as the expression of a host-derived RNA biomarker and classifies. Examples of machine learning models include neural networks, including recurrent neural networks and convolutional neural networks; random forests models, including random forests; restricted Boltzmann machines; recurrent tensor networks; and gradient boosted trees. The term "classifier" (or classification model) is sometimes used to describe all forms of classification model including deep learning models (e.g., neural networks having many layers) as well as random forests models.

As used herein, "quantify" means to identify the presence or quantity of an RNA biomarker from a sample.

As used herein, a machine learning system may include a deep learning model that may include a function approximation method aiming to develop custom dictionaries configured to achieve a given task, be it classification or dimension reduction. It may be implemented in various forms such as by a neural network (e.g., a convolutional neural network), etc. In general, though not necessarily, it includes multiple layers. Each such layer includes multiple processing nodes and the layers process in sequence, with nodes of layers closer to the model input layer processing before nodes of layers closer to the model output. In various embodiments, one-layer feeds to the next, etc. The output layer may include nodes that represent various classifications. In certain embodiments, machine learning systems may include artificial neural networks (ANNs) which are a type of computational system that can learn the relationships between an input data set and a target data set. ANN name originates from a desire to develop a simplified mathematical representation of a portion of the human neural system, intended to capture its "learning" and "generalization" abilities. ANNs are a major foundation in the field of artificial intelligence. ANNs are widely applied in research because they can model highly non-linear systems in which the relationship among the variables is unknown or very complex. ANNs are typically trained on empirically observed data sets. The data set may conventionally be divided into a training set, a test set, and a validation set.

Having now described the inventive technology, the same will be illustrated with reference to certain examples, which are included herein for illustration purposes only, and which are not intended to be limiting of the invention.

### EXAMPLES

### Example 1: Data Pre-Processing.

The present inventors processed the raw microarray or RNA sequencing data through standardized workflow. For Microarray datasets, the pipeline 1) performs background signal correction and signal normalization, 2) annotates probes on the microarray chip with known gene names and accession numbers, 3) filters probes based on the signal intensities. For RNA sequencing datasets, the pipeline 1) Filters out RNA-seq reads of low-quality and contaminating sequences 2) Maps the filtered reads to host (human) genome 3) Determines data quality based on trimming and mapping statistics 4) Assigns total number of RNA-seq reads mapped onto each annotated gene within human genome. This gene expression profile from both microarray and RNA sequencing datasets are indicative of the relative gene expression level. The pipeline may normalize the read counts based on a set of empirically-determined control genes and further conducts differential expression analysis to determine what are the significantly up-regulated genes within each study.

### Example 2: Biomarker Discovery.

Based on which host RNA biomarker is commonly upregulated across different pathogen infections, and how readily they can be detected across different cell types and tissue samples, the present inventors summarized the results from the above data pre-processing steps using statistical methods, including direct merge, combine p-value, combine effect size, combine ranks and/or co-expression analysis. These statistical measures combine the data in a way that accounts for confidence and reliability of the results.

Importantly, by focusing on studies that utilized similar infection data from broader categories (e.g. Domain level: virus, bacteria, etc; Viral class: herpesvirus, retrovirus, etc; Site of replication in the body: respiratory virus), the present inventors were also able to identify specific sets of host biomarkers that help differentiate the type of infection as explained below. These discovered biomarkers can either directly move on to empirical testing, or they can be further validated and prioritized by the computer-assisted approaches described in Example 3.

### Example 3: In silica Validation and Filtering.

In another embodiment, the invention may utilize a machine learning system. The summarized host biomarkers may optionally be subject to downstream validation and filtering *via* supervised machine-learning approaches. In one embodiment, the present inventors provided the classifier (Logistic regression, polynomial supported vector machine (SVM), Poisson linear discriminant or Convolutional Neuron Network ) with either the list of biomarkers or random genes (as control) to construct statistic models around training RNA-seq or RNA microarray datasets. Then the present inventors programmed the classifier to determine if a set of unknown RNA-seq or RNA microarray samples are infected. If the list of biomarkers helps predict the infection condition of the unknown data, the prediction accuracy would be significantly higher comparing to the control. To further utilize this approach to filter out less relevant biomarkers from the list, the present inventors removed individual genes from the biomarker list and carried out the entire classification iteratively. If the removal of that biomarker decreases the prediction accuracy, it suggests the biomarker being removed plays a key role in determining the infection condition. Reciprocally, if the removal of that biomarker increases, or has no effect on the prediction accuracy, the removed biomarker could be discarded due to its lack of relevancy.

### Example 4: Virus-specific Host Biomarkers RNA sequences.

One embodiment of the invention may include one or more of the following biomarkers, identified through the methods described herein, as being specifically upregulated in response to a viral infection in a human subject. In a preferred embodiment, the invention may include the early-detection of a viral infection in a host through the detection of one or more of the biomarkers according to SEQ ID NOs. 1-5. In one preferred embodiment, the invention may include the early-detection of a viral infection, such as SARS-CoV-2 (COVID-19 in a host through the detection of one or more of the biomarkers according to SEQ ID NOs. 1-5, the detection being accomplished, in one preferred embodiment, by a lateral flow device described by the present inventors in PCT Application No. PCT/US2020/049290, the specification and figures being incorporated herein by reference, or other biomarker detection systems known in the art. Additional embodiments for detecting one or more of the biomarkers identified herein may include a rapid detection LAMP assay, PCR, or other detection methods described generally herein and known in the art.

### Example 5: Bacteria-specific Host Biomarkers RNA sequences.

One embodiment of the invention may include one or more of the following biomarkers, identified through the methods described herein, as being specifically upregulated in response to a viral infection in a human subject. In a preferred embodiment, the invention may include the early-detection of a bacterial infection in a host through the detection of one or more of the biomarkers according to SEQ ID NOs. 6-10. In one preferred embodiment, the invention may include the early-detection of a bacterial infection in a host through the detection of one or more of the biomarkers according to SEQ ID NOs. 6-10, the detection being accomplished by a lateral flow device described by the present inventors in PCT Application No. PCT/US2020/049290, the specification and figures being incorporated herein by reference, or other biomarker detection systems known in the art. Additional embodiments for detecting one or more of the biomarkers identified herein may include a rapid detection LAMP assay, PCR, or other detection methods described generally herein and known in the art.

### Example 6: Retrovirus-specific Host Biomarkers RNA sequences.

One embodiment of the invention may include one or more of the following biomarkers, identified through the methods described herein, as being specifically upregulated in response to a viral infection in a human subject. In a preferred embodiment, the invention may include the early-detection of a retroviral infection in a host through the detection of one or more of the biomarkers according to SEQ ID NOs. 11-15. In one preferred embodiment, the invention may include the early-detection of a retroviral infection in a host through the detection of one or more of the biomarkers according to SEQ ID NOs. 11-15, the detection being accomplished by a lateral flow device described by the present inventors in PCT Application No. PCT/US2020/049290, the specification and figures being incorporated herein by reference, or other biomarker detection systems known in the art. Additional embodiments for detecting one or more of the biomarkers identified herein may include a rapid detection LAMP assay, PCR, or other detection methods described generally herein and known in the art.

### Example 7: Herpesvirus-specific Host Biomarkers RNA sequences.

One embodiment of the invention may include one or more of the following biomarkers, identified through the methods described herein, as being specifically upregulated in response to a viral infection in a human subject. In a preferred embodiment, the invention may include the early-detection of a herpesvirus infection in a host through the detection of one or more of the biomarkers according to SEQ ID NOs. 16-20. In one preferred embodiment, the invention may include the early-detection of a herpesvirus infection in a host through the detection of one or more of the biomarkers according to SEQ ID NOs. 16-20, the detection being accomplished by a lateral flow device described by the present inventors in PCT Application No. PCT/US2020/049290, the specification and figures being incorporated herein by reference, or other biomarker detection systems known in the art. Additional embodiments for detecting one or more of the biomarkers identified herein may include a rapid detection LAMP assay, PCR, or other detection methods described generally herein and known in the art.

### Example 8: Respiratory virus-specific Host Biomarkers RNA sequences.

One embodiment of the invention may include one or more of the following biomarkers, identified through the methods described herein, as being specifically upregulated in response to a viral infection in a human subject. In a preferred embodiment, the invention may include the early-detection of a respiratory infection, such as SARS-CoV-2 (COVID-19) in a host through the detection of one or more of the biomarkers according to SEQ ID NOs. 21-25. In one preferred embodiment, the invention may include the early-detection of a respiratory infection in a host through the detection of one or more of the biomarkers according to SEQ ID NOs. 21-25, the detection being accomplished by a lateral flow device described by the present inventors in PCT Application No. PCT/US2020/049290, the specification and figures being incorporated herein by reference, or other biomarker detection systems known in the art. Additional embodiments for detecting one or more of the biomarkers identified herein may include a rapid detection LAMP assay, PCR, or other detection methods described generally herein and known in the art.

### Example 9: Eukaryotic and/or Protist virus-specific Host Biomarkers RNA sequences.

One embodiment of the invention may include one or more of the following biomarkers, identified through the methods described herein, as being specifically upregulated in response to a eukaryotic or protist pathogen infection in a human subject. In a preferred embodiment, the invention may include the early-detection of a eukaryotic or protist pathogen infection, such as Plasmodium falciparum (P. falciparum), the causative agent of Malaria in a host through the detection of one or more of the biomarkers according to SEQ ID NOs. 26-30. In one preferred embodiment, the invention may include the early-detection of a eukaryotic or protist pathogen infection in a host through the detection of one or more of the biomarkers according to SEQ ID NOs. 26-30, the detection being accomplished by a lateral flow device described by the present inventors in PCT Application No. PCT/US2020/049290, the specification and figures being incorporated herein by reference, or other biomarker detection systems known in the art. Additional embodiments for detecting one or more of the biomarkers identified herein may include a rapid detection LAMP assay, PCR, or other detection methods described generally herein and known in the art.
The following numbered examples are provided to further illustrate the invention described herein:
Example 1. Use of one or more of the nucleotide sequences identified in SEQ ID NOs. 1-30, and their corresponding encoded mRNA transcript and/or translated polypeptide as biomarkers for early-infection in a subject or for identification of pathogen class-specific infection in a subject. Example 2. Method for diagnosing early-infection in a subject comprising determining the presence or expression levels of the biomarker in a biological sample obtained from said patient, wherein the presence or expression levels of the biomarker selected from the group consisting of: the nucleotide sequences according to SEQ ID NOs. 1-30, are indicative of infection or possible infection by one or more pathogens.
Example 3. The use of example 1 or the method of example 2, wherein said sequence is associated with a viral infection and is selected from the group consisting of: the nucleotide sequences according to SEQ ID NOs. 1-5.
Example 4. The use of example 1 or the method of example 2, wherein said sequence is associated with a bacterial infection and is selected from the group consisting of: the nucleotide sequences according to SEQ ID NOs. 6-10.
Example 5. The use of example 1 or the method of example 2, wherein said sequence is associated with a retroviral infection and is selected from the group consisting of: the nucleotide sequences according to SEQ ID NOs. 11-15.
Example 6. The use of example 1 or the method of example 2, wherein said sequence is sequence associated with a herpesvirus infection and is selected from the group consisting of: the nucleotide sequences according to SEQ ID NOs. 16-20.
Example 7. The use of example 1 or the method of example 2, wherein said sequence is sequence associated with a respiratory infection and is selected from the group consisting of: the nucleotide sequences according to SEQ ID NOs. 21-25.
Example 8. The use of example 1 or the method of example 2, wherein said sequence is associated with a eukaryotic pathogen infection and is selected from the group consisting of: the nucleotide sequences according to SEQ ID NOs. 26-30.
Example 9. The use of example 1 or the method of example 2, wherein the pathogen comprises a pathogen selected from the group consisting of:
   - a viral pathogen;
   - a bacterial pathogen;
   - a eukaryotic pathogen;
   - a protist pathogen;
   - a respiratory pathogen;
   - a blood pathogen; and
   - a gastrointestinal pathogen.
   - a retrovirus pathogen; and
   - a herpesvirus pathogen.
Example 10. The use of example 1 or the method of example 2, wherein said sequence is defined by SEQ ID NO. 2, SEQ ID NO. 4, SEQ ID NO. 5, SEQ ID NO. 3, SEQ ID NO. 21 and/or SEQ ID NO. 28.
Example 11. One or more of the host-biomarkers having nucleotide sequences identified in: SEQ ID NOs. 1-30 for use in identification of the site of replication, or infection in a subject.
Example 12. One or more of the host-biomarkers for use according to example 10, wherein said biomarkers are defined by SEQ ID NO. 2, SEQ ID NO. 4, SEQ ID NO. 5, SEQ ID NO. 3, SEQ ID NO. 21 and/or SEQ ID NO. 28.

### TABLES

### TABLE 1: Exemplary Host Biomarker identification

| | |
|---|---|
| SEQ ID NO. 1: | indoleamine 2,3-dioxygenase 1 (IDO1) (mRNA) |
| SEQ ID NO. 2: | interferon induced protein with tetratricopeptide repeats 2 (IFIT2), (mRNA) |
| SEQ ID NO. 3: | guanylate binding protein 4 (GBP4), (mRNA) |
| SEQ ID NO. 4: | ISG15 ubiquitin like modifier (ISG15), (mRNA) |
| SEQ ID NO. 5: | radical S-adenosyl methionine domain containing 2 (RSAD2), (mRNA) |
| SEQ ID NO. 6: | methionine adenosyltransferase 1A (MAT1A), (mRNA) |
| SEQ ID NO. 7: | caspase 16, pseudogene (CASP16P), (non-coding RNA) |
| SEQ ID NO. 8: | U1 small nuclear 2 (RNU1-2), (small nuclear RNA) |
| SEQ ID NO. 9: | ArfGAP with GTPase domain, ankyrin repeat and PH domain 11 (AGAP11), (mRNA) |
| SEQ ID NO. 10: | synaptotagmin 4 (SYT4), (mRNA) |
| SEQ ID NO. 11: | glutaminyl-peptide cyclotransferase (QPCT), (mRNA) |
| SEQ ID NO. 12: | interleukin 2 (IL2), (mRNA) |
| SEQ ID NO. 13: | brain abundant membrane attached signal protein 1 (BASP1), transcript variant 1, (mRNA) |
| SEQ ID NO. 14: | family with sequence similarity 30 member A (FAM30A), (long non-coding RNA) |
| SEQ ID NO. 15: | tetraspanin 13 (TSPAN13), (mRNA) |
| SEQ ID NO. 16: | WWC2 antisense RNA 2 (WWC2-AS2), (long non-coding RNA) |
| SEQ ID NO. 17: | prothymosin alpha (PTMA), transcript variant X5, (mRNA) |
| SEQ ID NO. 18: | zinc finger protein 296 (ZNF296), (mRNA) |
| SEQ ID NO. 19: | F-box and WD repeat domain containing 4 pseudogene 1 (FBXW4P1), (non-coding RNA) |
| SEQ ID NO. 20: | SRY-box transcription factor 3 (SOX3), (mRNA) |
| SEQ ID NO. 21: | C-C motif chemokine ligand 8 (CCL8), (mRNA) |
| SEQ ID NO. 22: | cytochrome P450 family 1 subfamily B member 1 (CYP1B1), (mRNA) |
| SEQ ID NO. 23: | long intergenic non-protein coding RNA 2057 (LINC02057), (long non-coding RNA) |
| SEQ ID NO. 24: | adrenoceptor alpha 2B (ADRA2B), (mRNA) |
| SEQ ID NO. 25: | UDP-GlcNAc:betaGal beta-1,3-N-acetylglucosaminyltransferase 6 (B3GNT6), (mRNA) |
| SEQ ID NO. 26: | ankyrin repeat domain 22 (ANKRD22), (mRNA) |
| SEQ ID NO. 27: | FERM domain containing 3 (FRMD3), transcript variant 1, (mRNA) |
| SEQ ID NO. 28: | leucine aminopeptidase 3 (LAP3), (mRNA) |
| SEQ ID NO. 29: | syntaxin 11 (STX11), (mRNA) |
| SEQ ID NO. 30: | toll like receptor 7 (TLR7), (mRNA) |

Below the disclosure will be summarized as follows:
1. A method of identifying general host-derived RNA biomarkers of infection comprising the steps of:
   a) introducing a first pathogen to a biological sample;
   b) quantifying RNA transcripts from said biological sample;
   c) quantifying RNA transcripts from one or more negative control biological sample;
   d) identifying RNA transcripts that are upregulated in response to said first pathogen;
   e) optionally repeating steps, a-d using one or more additional pathogens to generate an RNA transcript expression data set; and
   f) analyzing said RNA transcript expression data set and identifying general host-derived RNA biomarkers of infection that are commonly upregulated in response to the pathogen infections.
2. A method of identifying host-derived RNA biomarkers of infection comprising the steps of:
   a) introducing a first bacterial pathogen to a first biological sample;
   b) introducing a first viral pathogen to a second biological sample;
   c) quantifying RNA transcripts from said first and second biological samples;
   d) quantifying RNA transcripts from a negative control biological sample corresponding to said first and second biological samples;
   e) identifying RNA transcripts from said first and second biological samples that are upregulated in response to said first bacterial pathogen and said first viral pathogen;
   f) optionally repeating steps, a-e using one or more additional viral and bacterial pathogens to generate an RNA transcript expression data set; and
   g) analyzing said RNA transcript expression data set and identifying host-derived RNA biomarkers of infection that are differentially upregulated in response to the viral and bacterial pathogen infections.
3. A method of identifying and differentiating host-derived RNA biomarkers of infection comprising the steps of:
   a) introducing a first pathogen to a first biological sample;
   b) introducing a second pathogen to a second biological sample;
   c) quantifying RNA transcripts from said first and second biological samples;
   d) quantifying RNA transcripts from a negative control biological sample corresponding to said first and second biological samples;
   e) identifying RNA transcripts from said first and second biological samples that are upregulated in response to said first pathogen and said second pathogen;
   f) optionally repeating steps, a-e using one or more additional pathogens to generate a data set; and
   g) analyzing said RNA transcript expression data set and identifying host-derived RNA biomarkers of infection that are differentially upregulated in response to the pathogen infections.
4. The method of any of clauses 1-3 wherein the pathogen comprises a pathogen selected from the group consisting of:
   - a viral pathogen;
   - a bacterial pathogen;
   - a eukaryotic pathogen;
   - a protist pathogen;
   - a respiratory pathogen;
   - a blood pathogen; and
   - a gastrointestinal pathogen.
   - a retrovirus pathogen; and
   - a herpesvirus pathogen.
5. The method of any of clauses 1-3 wherein the host-derived RNA biomarker is selected from the group consisting of: nucleotide sequence according to SEQ ID NO. 1-30.
6. The method of any of clauses 1-3 wherein said host-derived RNA biomarker sequence comprises a host-derived RNA biomarker sequence associated with a viral infection and is selected from the group consisting of: the nucleotide sequences according to SEQ ID NOs. 1-5.
7. The method of clause 4 wherein said host-derived RNA biomarker sequence comprises a host-derived RNA biomarker sequence associated with a bacterial infection and is selected from the group consisting of: the nucleotide sequences according to SEQ ID NOs. 6-10.
8. The method of clause 4 wherein said host-derived RNA biomarker sequence comprises a host-derived RNA biomarker sequence associated with a retroviral infection and is selected from the group consisting of: the nucleotide sequences according to SEQ ID NOs. 11-15.
9. The method of clause 4 wherein said host-derived RNA biomarker sequence comprises a host-derived RNA biomarker sequence associated with a herpesvirus infection and is selected from the group consisting of: the nucleotide sequences according to SEQ ID NOs. 16-20.
10. The method of clause 4 wherein said host-derived RNA biomarker sequence comprises a host-derived RNA biomarker sequence associated with a respiratory infection and is selected from the group consisting of: the nucleotide sequences according to SEQ ID NOs. 21-25.
11. The method of clause 4 wherein said host-derived RNA biomarker sequence comprises a host-derived RNA biomarker sequence associated with a eukaryotic pathogen infection and is selected from the group consisting of: the nucleotide sequences according to SEQ ID NOs. 26-30.
12. The method of any of clauses 1-3 wherein the biological sample comprises a biological sample from a human.
13. A method of identifying host-derived biomarkers of infection comprising the steps of:
   - generating a raw dataset of host-derived biomarker sequence reads;
   - performing data pre-processing on said raw dataset of host biomarker sequence reads comprising one or more of the following steps:
      - filtering out low quality biomarker sequence reads;
      - filtering out contaminating biomarker sequence reads;
      - mapping the filtered biomarker sequence reads to a reference genome, and optionally a human genome;
      - assigning total number of biomarker sequence reads mapped onto each annotated gene within said reference genome;
      - normalizing the biomarker sequence reads counts based on one or more control genes;
      - conducting differential expression analysis to determine which host biomarker genes are up-regulated in the dataset; and
   - outputting a dataset of upregulated host-derived biomarkers sequences.
14. The method of clause 13 and further comprising the steps of:
   - merging a plurality of datasets of upregulated host-derived biomarkers sequences for analysis and categorization comprising one or more of the following steps:
      - directly merging said plurality of datasets of upregulated host-derived biomarkers sequences;
      - combining the P-value of said plurality of datasets of upregulated host-derived biomarkers sequences;
      - combining the effect size of said plurality of datasets of upregulated host-derived biomarkers sequences;
      - combining the rank of said plurality of datasets of upregulated host-derived biomarkers sequences;
      - conduct co-expression and network analysis of said plurality of datasets of upregulated host-derived biomarkers sequences; and
   - outputting a dataset of ranked host-derived biomarkers sequences.
15. The method of clause 14 and further comprising the steps of:
   - validating said dataset of ranked host-derived biomarkers sequences comprising one or more of the following steps:
      - comparing a dataset of random gene controls against said dataset of ranked host-derived biomarkers sequences using a machine learning system comprising a classifier;
      - conducting cross-validation on said dataset being applied to said classifier to predict infection or non-infected states of a dataset of unknown RNA sequences; and
   - outputting a dataset of ranked and filtered host-derived biomarker sequences.
16. The method of any of clauses 13-15 wherein the host-derived biomarker sequences comprises host-derived RNA biomarker sequences.
17. The method of any of clauses 15, and 16 wherein said ranked and filtered host-derived biomarker sequences comprises ranked and filtered host-derived RNA biomarker sequences.
18. The method of clause 17 wherein said ranked and filtered host-derived RNA biomarker sequences are ranked and filtered according to one or more of the following:
   - a host-derived RNA biomarker sequence associated with a viral infection;
   - a host-derived RNA biomarker sequence associated with a bacterial infection;
   - a host-derived RNA biomarker sequence associated with a eukaryotic pathogen infection;
   - a host-derived RNA biomarker sequence associated with a protist infection;
   - a host-derived RNA biomarker sequence associated with a respiratory infection;
   - a host-derived RNA biomarker sequence associated with a blood infection; and
   - a host-derived RNA biomarker sequence associated with a gastrointestinal infection.
19. The method of clause 18 wherein said host-derived RNA biomarker sequence associated with a viral infection comprises a host-derived RNA biomarker sequence associated with a viral infection selected from the group consisting of:
   - a host-derived RNA biomarker sequence associated with a retroviral infection; and
   - a host-derived RNA biomarker sequence associated with a SARS-CoV-2 infection.
   - a host-derived RNA biomarker sequence associated with a herpesvirus infection.
20. The method of clause 17 wherein said ranked and filtered host-derived RNA biomarker sequence is selected from the group consisting of: the nucleotide sequences according to SEQ ID NOs. 1-30.
21. The method of clause 18 wherein said host-derived RNA biomarker sequence associated with a viral infection is selected from the group consisting of: the nucleotide sequences according to SEQ ID NOs. 1-5.
22. The method of clause 18 wherein said host-derived RNA biomarker sequence associated with a bacterial infection is selected from the group consisting of: the nucleotide sequences according to SEQ ID NOs. 6-10.
23. The method of clause 18 wherein said host-derived RNA biomarker sequence associated with a retroviral infection is selected from the group consisting of: the nucleotide sequences according to SEQ ID NOs. 11-15.
24. The method of clause 18 wherein said host-derived RNA biomarker sequence associated with a herpesvirus infection is selected from the group consisting of: the nucleotide sequences according to SEQ ID NOs. 16-20.
25. The method of clause 18 wherein said host-derived RNA biomarker sequence associated with a respiratory infection is selected from the group consisting of: the nucleotide sequences according to SEQ ID NOs. 21-25.
26. The method of clause 18 wherein said host-derived RNA biomarker sequence associated with a eukaryotic pathogen infection and is selected from the group consisting of: the nucleotide sequences according to SEQ ID NOs. 26-30.
27. The method of any of clauses 13-15 wherein the host-derived biomarker sequences are validated *in vivo.*
28. The method of any of clauses 13-15, and 27 and wherein the host-derived biomarker sequences are validated in a human subject.
29. A method of identifying host-derived biomarkers of infection comprising the steps of:
   - generating one or more microarray raw datasets of host-derived biomarkers wherein said raw data sets are derived from quantitative microarray data;
   - performing data pre-processing step on said microarray raw dataset of host biomarkers comprising one or more of the following steps:
      - correcting for background signals in the microarray raw dataset;
      - normalizing the signal for host-derived biomarkers in the microarray raw dataset,
      - annotating probes on the microarray chip with known gene names and accession numbers,
      - filtering host-derived biomarkers probes based on the signal intensity;
      - normalizing the host-derived biomarkers gene expression based on one or more control genes;
      - conducting differential expression analysis to determine which host biomarker genes are up-regulated in the dataset; and
   - outputting a dataset of upregulated host-derived biomarkers sequences.
30. The method of clause 29 and further comprising the steps of:
   - merging a plurality of datasets of upregulated host-derived biomarkers sequences for analysis and categorization comprising one or more of the following steps:
      - directly merging said plurality of datasets of upregulated host-derived biomarkers sequences;
      - combining the P-value of said plurality of datasets of upregulated host-derived biomarkers sequences;
      - combining the effect size of said plurality of datasets of upregulated host-derived biomarkers sequences;
      - combining the rank of said plurality of datasets of upregulated host-derived biomarkers sequences;
      - conduct co-expression and network analysis of said plurality of datasets of upregulated host-derived biomarkers sequences; and
   - outputting a dataset of ranked host-derived biomarkers sequences.
31. The method of clause 30 and further comprising the steps of:
   - validating said dataset of ranked host-derived biomarkers sequences comprising one or more of the following steps:
      - comparing a dataset of random gene controls against said dataset of ranked host-derived biomarkers sequences using machine learning system comprising a classifier;
      - conducting cross-validation on said dataset being applied to said classifier to predict infection or non-infected states of a dataset of unknown RNA sequences; and
   - outputting a dataset of ranked and filtered host-derived biomarker sequences.
32. The method of any of clauses 29-31 wherein the host-derived biomarker sequences comprises host-derived RNA biomarker sequences.
33. The method of any of clauses 31-32 wherein said ranked and filtered host-derived biomarker sequences comprises ranked and filtered host-derived RNA biomarker sequences.
34. The method of clause 33 wherein said ranked and filtered host-derived RNA biomarker sequences are ranked and filtered according to one or more of the following:
   - a host-derived RNA biomarker sequence associated with a viral infection;
   - a host-derived RNA biomarker sequence associated with a bacterial infection;
   - a host-derived RNA biomarker sequence associated with a eukaryotic pathogen infection;
   - a host-derived RNA biomarker sequence associated with a protist infection;
   - a host-derived RNA biomarker sequence associated with a respiratory infection;
   - a host-derived RNA biomarker sequence associated with a blood infection; and
   - a host-derived RNA biomarker sequence associated with a gastrointestinal infection.
35. The method of clause 34 wherein said host-derived RNA biomarker sequence associated with a viral infection comprises a host-derived RNA biomarker sequence associated with a viral infection selected from the group consisting of:
   - a host-derived RNA biomarker sequence associated with a retroviral infection; and
   - a host-derived RNA biomarker sequence associated with a herpesvirus infection.
36. The method of clause 34 wherein said ranked and filtered host-derived RNA biomarker sequence is selected from the group consisting of: the nucleotide sequences according to SEQ ID NOs. 1-30.
37. The method of clause 34 wherein said host-derived RNA biomarker sequence associated with a viral infection is selected from the group consisting of: the nucleotide sequences according to SEQ ID NOs. 1-5.
38. The method of clause 34 wherein said host-derived RNA biomarker sequence associated with a bacterial infection is selected from the group consisting of: the nucleotide sequences according to SEQ ID NOs. 6-10.
39. The method of clause 34 wherein said host-derived RNA biomarker sequence associated with a retroviral infection is selected from the group consisting of: the nucleotide sequences according to SEQ ID NOs. 11-15.
40. The method of clause 34 wherein said host-derived RNA biomarker sequence associated with a herpesvirus infection is selected from the group consisting of: the nucleotide sequences according to SEQ ID NOs. 16-20.
41. The method of clause 34 wherein said host-derived RNA biomarker sequence associated with a respiratory infection is selected from the group consisting of: the nucleotide sequences according to SEQ ID NOs. 21-25.
42. The method of clause 34 wherein said host-derived RNA biomarker sequence associated with a eukaryotic pathogen infection and is selected from the group consisting of: the nucleotide sequences according to SEQ ID NOs. 26-30.
43. The method of clauses 29-31 wherein the host-derived biomarker sequences are validated *in vivo.*
44. The method of clauses 29-31, and 43 and wherein the host-derived biomarker sequences are validated in a human subject.
45. A method of identifying host-derived biomarkers of infection comprising the steps of:
   - generating one or more raw datasets of host-derived biomarkers wherein said raw data sets are derived from quantitative real time PCR (qRT-PCR) datasets;
   - performing data pre-processing step on said raw dataset of host biomarkers comprising one or more of the following steps:
      - filtering out low quality biomarker sequence reads;
      - filtering out contaminating biomarker sequence reads;
      - mapping the filtered biomarker sequence reads to a reference genome, and optionally a human genome;
      - assigning total number of biomarker sequence reads mapped onto each annotated gene within said reference genome;
      - normalizing the biomarker sequence reads counts based on one or more control genes;
      - conducting differential expression analysis to determine which host biomarker genes are up-regulated in the dataset; and
   - outputting a dataset of upregulated host-derived biomarkers sequences.
46. The method of clause 45 and further comprising the steps of:
   - merging a plurality of datasets of upregulated host-derived biomarkers sequences for analysis and categorization comprising one or more of the following steps:
      - directly merging said plurality of datasets of upregulated host-derived biomarkers sequences;
      - combining the P-value of said plurality of datasets of upregulated host-derived biomarkers sequences;
      - combining the effect size of said plurality of datasets of upregulated host-derived biomarkers sequences;
      - combining the rank of said plurality of datasets of upregulated host-derived biomarkers sequences;
      - conduct co-expression and network analysis of said plurality of datasets of upregulated host-derived biomarkers sequences; and
   - outputting a dataset of ranked host-derived biomarkers sequences.
47. The method of clause 46 and further comprising the steps of:
   - validating said dataset of ranked host-derived biomarkers sequences comprising one or more of the following steps:
      - comparing a dataset of random gene controls against said dataset of ranked host-derived biomarkers sequences using machine learning system comprising a classifier;
      - conducting cross-validation on said dataset being applied to said classifier to predict infection or non-infected states of a dataset of unknown RNA sequences; and
   - outputting a dataset of ranked and filtered host-derived biomarker sequences.
48. The method of any of clauses 45-47 wherein the host-derived biomarker sequences comprises host-derived RNA biomarker sequences.
49. The method of any of clauses 47-48 wherein said ranked and filtered host-derived biomarker sequences comprises ranked and filtered host-derived RNA biomarker sequences.
50. The method of clause 49 wherein said ranked and filtered host-derived RNA biomarker sequences are ranked and filtered according to one or more of the following:
   - a host-derived RNA biomarker sequence associated with a viral infection;
   - a host-derived RNA biomarker sequence associated with a bacterial infection;
   - a host-derived RNA biomarker sequence associated with a respiratory infection;
   - a host-derived RNA biomarker sequence associated with a eukaryotic pathogen infection;
   - a host-derived RNA biomarker sequence associated with a protist infection;
   - a host-derived RNA biomarker sequence associated with a blood infection; and
   - a host-derived RNA biomarker sequence associated with a gastrointestinal infection.
51. The method of clause 50 wherein said host-derived RNA biomarker sequence associated with a viral infection comprises a host-derived RNA biomarker sequence associated with a viral infection selected from the group consisting of:
   - a host-derived RNA biomarker sequence associated with a retroviral infection; and
   - a host-derived RNA biomarker sequence associated with a herpesvirus infection.
52. The method of clause 50 wherein said ranked and filtered host-derived RNA biomarker sequence is selected from the group consisting of: the nucleotide sequences according to SEQ ID NOs. 1-30.
53. The method of clause 50 wherein said host-derived RNA biomarker sequence associated with a viral infection is selected from the group consisting of: the nucleotide sequences according to SEQ ID NOs. 1-5.
54. The method of clause 50 wherein said host-derived RNA biomarker sequence associated with a bacterial infection is selected from the group consisting of: the nucleotide sequences according to SEQ ID NOs. 6-10.
55. The method of clause 50 wherein said host-derived RNA biomarker sequence associated with a retroviral infection is selected from the group consisting of: the nucleotide sequences according to SEQ ID NOs. 11-15.
56. The method of clause 50 wherein said host-derived RNA biomarker sequence associated with a herpesvirus infection is selected from the group consisting of: the nucleotide sequences according to SEQ ID NOs. 16-20.
57. The method of clause 50 wherein said host-derived RNA biomarker sequence associated with a respiratory infection is selected from the group consisting of: the nucleotide sequences according to SEQ ID NOs. 21-25.
58. The method of clause 50 wherein said host-derived RNA biomarker sequence associated with a eukaryotic pathogen infection and is selected from the group consisting of: the nucleotide sequences according to SEQ ID NOs. 26-30.
59. The method of any of clauses 45-47 wherein the host-derived biomarker sequences are validated *in vivo.*
60. The method of any of clauses 45-47, and 59 and wherein the host-derived biomarker sequences are validated in a human subject.
61. A method of identifying general host-derived RNA biomarkers of infection comprising the steps of:
   - introducing a first pathogen to a subject;
   - quantifying RNA transcripts from said subject;
   - quantifying RNA transcripts from one or more negative control subjects ;
   - identifying RNA transcripts that are upregulated in response to said first pathogen;
   - optionally repeating the above four steps using one or more additional pathogens to generate an RNA transcript expression data set; and
   - analyzing said RNA transcript expression data set and identifying general host-derived RNA biomarkers of infection that are commonly upregulated in response to the pathogen infections.
62. A method of identifying host-derived RNA biomarkers of infection comprising the steps of:
   - introducing a first bacterial pathogen to a first subject;
   - introducing a first viral pathogen to a second subject;
   - quantifying RNA transcripts from said first and second subjects;
   - quantifying RNA transcripts from a negative control subject corresponding to said first and second subjects;
   - identifying RNA transcripts from said first and second subjects that are upregulated in response to said first bacterial pathogen and said first viral pathogen;
   - optionally repeating the above five steps using one or more additional viral and bacterial pathogens to generate an RNA transcript expression data set; and
   - analyzing said RNA transcript expression data set and identifying host-derived RNA biomarkers of infection that are differentially upregulated in response to the viral and bacterial pathogen infections.
63. A method of identifying and differentiating host-derived RNA biomarkers of infection comprising the steps of:
   - introducing a first pathogen to a first subject;
   - introducing a second pathogen to a second subject;
   - quantifying RNA transcripts from said first and second subjects;
   - quantifying RNA transcripts from a negative control subject corresponding to said first and second subjects;
   - identifying RNA transcripts from said first and second subjects that are upregulated in response to said first pathogen and said second pathogen;
   - optionally repeating the above five steps using one or more additional pathogens to generate a data set; and
   - analyzing said RNA transcript expression data set and identifying host-derived RNA biomarkers of infection that are differentially upregulated in response to the pathogen infections.
64. The method of clauses 53-54, and 55 wherein the pathogen comprises a pathogen selected from the group consisting of:
   - a viral pathogen;
   - a bacterial pathogen;
   - a eukaryotic pathogen;
   - a protist infection;
   - a respiratory pathogen;
   - a blood pathogen; and
   - a gastrointestinal pathogen.
   - a retrovirus pathogen; and
   - a herpesvirus pathogen.
65. The method of clauses 61-63 wherein the host-derived RNA biomarker is selected from the group consisting of: nucleotide sequence according to SEQ ID NO. 1-30.
66. The method of clauses 61-63 wherein said host-derived RNA biomarker sequence comprises a host-derived RNA biomarker sequence associated with a viral infection and is selected from the group consisting of: the nucleotide sequences according to SEQ ID NOs. 1-5.
67. The method of clauses 64 wherein said host-derived RNA biomarker sequence comprises a host-derived RNA biomarker sequence associated with a bacterial infection and is selected from the group consisting of: the nucleotide sequences according to SEQ ID NOs. 6-10.
68. The method of clauses 64 wherein said host-derived RNA biomarker sequence comprises a host-derived RNA biomarker sequence associated with a retroviral infection and is selected from the group consisting of: the nucleotide sequences according to SEQ ID NOs. 11-15.
69. The method of clauses 64 wherein said host-derived RNA biomarker sequence comprises a host-derived RNA biomarker sequence associated with a herpes infection and is selected from the group consisting of: the nucleotide sequences according to SEQ ID NOs. 16-20.
70. The method of clauses 64 wherein said host-derived RNA biomarker sequence comprises a host-derived RNA biomarker sequence associated with a respiratory infection and is selected from the group consisting of: the nucleotide sequences according to SEQ ID NOs. 21-25.
71. The method of clauses 64 wherein said host-derived RNA biomarker sequence comprises a host-derived RNA biomarker sequence associated with a respiratory infection and is selected from the group consisting of: the nucleotide sequences according to SEQ ID NOs. 26-30.
72. The method of any of clauses 61-63 wherein the subject comprises a human subject participating in a clinical trial.
73. A method comprising:
   - identifying one or more RNA biomarkers of infection;
   - correlating said biomarker RNA biomarkers of infection with an infection type selected from the group consisting of:
      - a viral infection;
      - a bacterial infection;
      - a eukaryotic infection;
      - a protist infection;
      - a respiratory virus infection;
      - a blood infection; and
      - a gastrointestinal infection.
      - a retrovirus infection; and
      - a herpesvirus infection.
74. A method comprising:
   - identifying one or more RNA biomarkers of infection;
   - correlating said biomarker RNA biomarkers of infection with the pathogen causing said infection selected from the group consisting of:
      - a viral pathogen;
      - a bacterial pathogen;
      - a eukaryotic pathogen;
      - a protist pathogen;
      - a respiratory pathogen;
      - a blood pathogen; and
      - a gastrointestinal pathogen.
      - a retrovirus pathogen; and
      - a herpesvirus pathogen.
75. A method comprising:
   - identifying one or more RNA biomarkers of infection;
   - correlating said biomarker RNA biomarkers of infection with the pathogen causing said infection selected from the group consisting of: dengue virus type 2 (DENV2); influenza A virus (IAV) ; herpes simplex virus (HSV); human rhinovirus (HRV); respiratory syncytial virus (RSV); SARS-CoV-2 (COVID-19); *Staph aureus;* and P. falciparum.
76. The method of any of the above clauses wherein said pathogen is selected from the group consisting of: dengue virus type 2 (DENV2); influenza A virus (IAV) ; herpes simplex virus (HSV); human rhinoviruss (HRV); respiratory syncytial virus (RSV); SARS-CoV-2 (COVID-19); Staph aureus; and P. falciparum.
77. The method of any of the above clauses wherein said RNA biomarker of infection is selected from the group consisting of: C3, CXCL5, DDX58, EGR1, ICAM1, IRAK2, NFKBIA, PMAIP1, PPP1R15A, ZFP36, GBP4, IDO1, IFIT2, ISG15, RSAD2, MAT1A, CASP16P, RNU1-2, AGAP11, SYT4, ANKRD22, FRMD3, LAP3, STX11, TLR7, QPCT, IL2, BASP1, FAM30A, WWC2-AS, PTMA, ZNF296, FBXW4P1, SOX3, CCL8, CYP1B1, LINC0205, ADRA2B, and B3GNT6.

## Claims

1. Use of one or more of the nucleotide sequences identified in SEQ ID NOs. 2 and 5, and their corresponding encoded mRNA transcript and/or translated polypeptide as universal biomarkers for early-infection in a pre-symptomatic subject, wherein the biomarkers are upregulated in response to a viral, bacterial, or fungal infection.

2. Use of one or more of the mRNA transcripts selected from RSAD2 or IFIT2 as universal biomarkers for early-infection in a pre-symptomatic subject, wherein the biomarkers are upregulated in response to a viral, bacterial, or fungal infection.
